(19) Europäisches Patentamt
European Patent Office
Office européen des brevets

(11) EP 4 544 998 A1

(12) EUROPEAN PATENT APPLICATION

(43) Date of publication:
30.04.2025 Bulletin 2025/18

(21) Application number: 24208805.2

(22) Date of filing: 24.10.2024

(51) International Patent Classification (IPC):
A61B 5/08 $^{(2006.01)}$  A61B 5/00 $^{(2006.01)}$

(52) Cooperative Patent Classification (CPC):
A61B 5/0816; A61B 5/7207; A61B 5/7257;
A61B 5/086

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 26.10.2023 US 202363545906 P

(71) Applicant: Draeger Medical Systems, Inc.
Andover, MA 01810 (US)

(72) Inventor: ZUZARTE, Ian Jeromino
Chelmsford, 01824 (US)

(74) Representative: Haseltine Lake Kempner LLP
One Portwall Square
Portwall Lane
Bristol BS1 6BH (GB)

(54) **NELSON CROSS SPECTRAL METHOD FOR IMPEDANCE RESPIRATION RATE MEASUREMENT**

(57) A method for computing respiration rate and detecting events in a respiration signal representing the respiration of a monitored patient includes: performing a frequency domain analysis of the respiration signal to identify low frequency portions of the respiration signal that may be caused by one or more artifacts; and performing a time domain analysis to modify thresholds during artifacts to compute respiration rate from the time domain respiration signal. The method may be performed by a physiological monitoring device or a patient monitoring system employing a physiological monitoring device.

FIG. 1

EP 4 544 998 A1

**Description**

TECHNICAL FIELD

The present disclosure relates generally to the field of medical monitoring of physiological parameters in a patient and, more particularly, to monitoring a patient's condition using an electrocardiogram ("ECG").

BACKGROUND

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Conventional, modern medical practice frequently includes measurement and/or monitoring of a patient's physiological condition. Commonly known physiological parameters that are measured and/or monitored might include, for example and without limitation, blood pressure, body temperature, vision acuity, quantities of various substances in the blood, etc. Because of the value imparted by this information, the art of medical monitoring has developed an array of instruments and procedures for acquiring this kind of information.

One well-known measurement/monitoring technique is an "electrocardiogram" ("ECG"). Electrocardiograms are commonly used to monitor patients' heart conditions as well as to detect or predict cardiac events and conditions. In clinical settings, ECG signals representative of a patient's condition are captured in waveforms and analyzed by physiological monitoring devices. However, ECGs are also useful for acquiring other kinds of medical information besides cardiac events and conditions.

An ECG graphs voltage acquired from a person's body over time. The voltages represent electrical activity of the heart that cause the heart to beat. To acquire the voltages, sensors, or "electrodes", are placed at selected points on the person's body. The number and location of the electrodes are fairly standardized but may vary depending on the type of medical information that is of interest. For example, in one common nomenclature, the sensors are electrically connected to the physiological monitoring device by cables. The sensors and cables together are then referred to as "leads". The electrical leads may be, again without limitation, limb leads, augmented limb leads, precordial (or chest) leads, or some combination of these depending on the ECG procedure to be performed.

SUMMARY

Signals acquired in a patient monitoring context may be analyzed to determine physiological parameters of the patient that may reflect the patient's condition. The signals may be acquired through, for instance, an electrocardiograph ("ECG"). These signals are acquired over time-that is, in the time domain. However, the signals may, and typically do, include artifacts and other anomalies that interfere with an accurate determination of the physiological parameter. The present disclosure presents a technique by which the acquired time domain respiration signal is subjected to a frequency domain analysis to identify one or more artifacts or anomalies, that can then be filtered from the time domain respiration signal.

More particularly, a first embodiment is a method for use in quantifying a physiological parameter of a monitored patient. The method comprises performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

A second embodiment is a physiological monitoring device. The physiological monitoring device comprises a processor; and a memory encoded with instructions that, when executed by the processor, performs a method. The method comprises performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

A third embodiment is a patient monitoring system. The patient monitoring system comprises a plurality of electrocardiogram ("ECG") leads over which a time domain respiration signal representative of a physiological parameter may be obtained; and a physiological monitoring device. The physiological monitoring device comprises a processor; and a memory encoded with instructions that, when executed by the processor, performs a method. The method comprises performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

In a fourth embodiment, a method for use in monitoring the physical condition of a patient is substantially as shown and described.

In a fifth embodiment, a physiological monitoring device is substantially as shown and described.

In a sixth embodiment, a patient monitoring system is substantially as shown and described.

The above presents a simplified summary of the invention as claimed below in order to provide a basic understanding of some aspects of the invention. This summary is not an exhaustive overview of the invention. It is not intended to identify key or critical elements of the invention or to delineate the scope of the invention. Its sole purpose is to present some concepts in a simplified form as a prelude to the more detailed description that is discussed later.

BRIEF DESCRIPTION OF THE DRAWINGS

In the drawings, like reference numbers generally indicate identical, functionally similar, and/or structurally similar elements.

FIG. 1 illustrates a patient monitoring system according to one or more examples.

FIG. 2 is a schematic diagram of an example of a physiological monitoring device capable of executing a customizable physiological measurement schedule for measuring physiological parameters according to an embodiment of the present disclosure.

FIG. 3 is a schematic diagram of an example of a patient monitoring system including a computing system and the physiological monitoring system of FIG. 1 according to an embodiment of the present disclosure.

FIG. 4 presents a patient model illustrating these measurement and acquisition of the impedance respiration signals in the embodiments disclosed herein.

FIG. 5A-FIG. 5B illustrate a method for quantifying a physiological parameter of a monitored patient in accordance with one or more embodiments.

FIG. 6 schematically depicts one particular implementation of a physiological monitoring device.

FIG. 7 is an example waveform illustration of movement artifacts on the impedance respiration waveforms and the false apnea alarms due to undetected breaths.

FIG. 8 illustrates some of the concepts associated with peak and valley detection of the illustrated embodiments.

FIG. 9 illustrates detection of breaths and valleys from the upwave/lowwave levels based on the adaptive thresholds.

FIG. 10 illustrates the respiration rate during regular breathing, pauses in breathing and after the end of apnea and detection of an apnea.

FIG. 11 illustrates breaths after movement artifacts are detected after threshold adjustment suppressing any false apnea alarms.

FIG. 12 illustrates a method in accordance with one or more embodiments as described above.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the spirit and scope of the appended claims.

DETAILED DESCRIPTION

Illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

The physiological monitoring system disclosed herein is used, in accordance with the present disclosure, to measure "impedance respiration" ("IR"). Impedance respiration is an indirect method for measuring a patient's respiration rate ("RR"), which is a useful parameter in assessing a patient's health. Typical ranges for respiration rate are between 18 bpm and 60 bpm for neonates and 12 - 20 bpm for adults. Generally, respiration rate values outside of these ranges imply the patient may be in critical condition. Low respiration rate is called bradypnea while high respiration rate is called tachypnea. Impedance respiration is mainly used to monitor post-surgery patients who are under anesthesia and neonates. It is particularly useful for premature neonates, who are prone to apnea events (lack of breathing), which may lead to death if not treated for immediately.

The technique utilizes the fact that the thoracic impedance of a person varies with time as a function of their breathing cycle. When inhaling, the air entering the lungs causes a small increase to thoracic impedance, while exhaling causes a small decrease. The peak-to-peak change in impedance due to breathing is usually between $0.3\,\Omega$ to $3\,\Omega$ for most patients. However, there is also a constant component to the thoracic impedance due to the air remaining always in the lungs, and the impedance of the bones, blood, etc. of the human body. This constant, or DC component, is in the order of $500\,\Omega$ to $2\,k\Omega$ for most patients.

Although the ECG technology and the measurement of impedance respiration is well established, challenges remain. Ideally, the impedance respiration signal is a sinusoidal signal whose frequency is the respiration rate. However, in practice analyzing the impedance respiration signal and extracting an accurate respiration rate from it is not always trivial.

For example, one challenge is the presence of cardiac artifacts in the impedance respiration signal. The pumping of the blood due to the function of the heart creates small but measurable changes in thoracic impedance. These so-called cardiac artifacts are of amplitude usually negligible compared to the impedance variations caused by breathing. However, in cases of shallow breathing these cardiac artifacts can be erroneously interpreted as breathing and cause false tachypnea alarms, often with a respiration rate close to the heart rate. The presence of cardiac artifacts can be particularly strong in neonates.

A second challenge is what are called motion or movement artifacts. Patient motion causes the skin-electrode impedance to vary with time, and such impedance variations can be erroneously interpreted as changes to impedance due to breathing, thus leading to false respiration readings and inaccurate respiration rates.

Another challenge is presented by apneas missed by caregivers due to noise or artifacts. Apnea is a life-threatening condition defined as a long pause in breathing and the respiration monitoring technique should sound an alarm to these events. As per the default settings an apnea alarm is sounded when there are no breaths detected for 15 seconds. Occasionally, noise or cardiac/motion artifacts can cause a breath to be detected during a real apneic event. This can cause the apnea alarm event to not be called and lead to a critical condition going undetected.

Yet another challenge is the detection of false apneas due to low impedance sensitivity. Missed apneic alarm can have fatal consequences. However, frequent false alarms can have similar consequences. High number of false alarms has led to alarm fatigue, a sensory overload when clinicians are exposed to an excessive number of alarms, resulting in desensitization to alarms and missed alarms. Patient deaths have been attributed to alarm fatigue. False apneic alarms can occur due to shallow breathing especially common in neonates. Shallow breathing could cause rapid transitions from normal impedance respiration to low impedance levels. Breaths during such low impedance should be accurately detected to avoid false alarms.

Those in the art having the benefit of this disclosure will appreciate that there may still be other challenges in the measurement of impedance respiration through ECG analysis. The implications of these and other challenges, as set forth above, can be quite serious. Accordingly, the presently disclosed technique is directed to resolving, or at least mitigating, these challenges.

The technique disclosed herein is disclosed in the context of one or more embodiments in which the impedance respiration is obtained in order to determine a patient's respiration rate. However, the technique is not limited to the ascertainment of impedance respiration. It is to be understood that the technique can be applied to quantify any patient physiological parameter that may be reflected in an ECG signal. Those in the art having the benefit of this disclosure will be able to readily adapt the disclosed technique to physiological parameters other than impedance respiration.

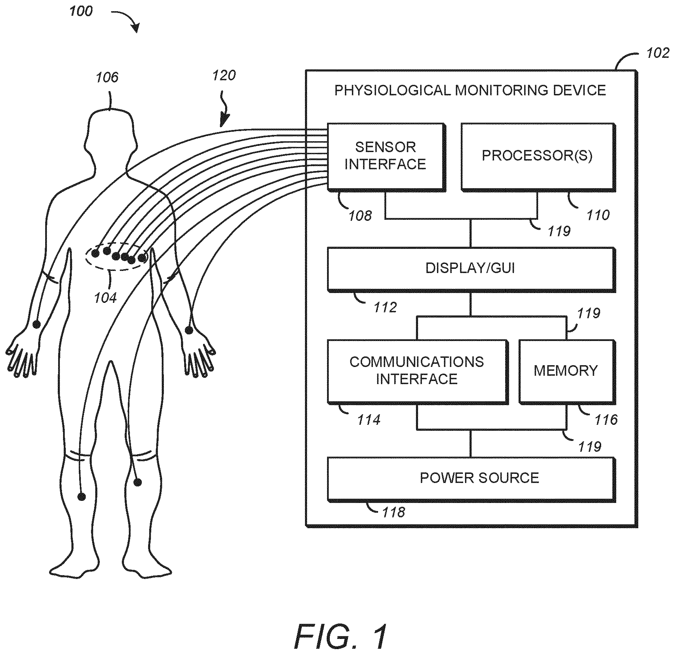

Turning now to the drawings, FIG. 1 illustrates a physiological monitoring system 100 according to one or more examples. As shown in FIG. 1, the system 100 includes a physiological monitoring device 102 capable of receiving physiological data from various sensors 104 connected to a patient 106 when deployed. In this example, the plurality of sensors 104 comprise electrocardiogram ("ECG") electrodes affixed to the skin of patient 106.

In general, it is contemplated by the present disclosure that patient monitor 102 includes electronic components and/or electronic computing devices operable to receive, transmit, process, store, and/or manage patient data and information associated performing the functions of the system as described herein, which encompasses any suitable processing device adapted to perform computing tasks consistent with the execution of computer-readable instructions stored in a memory or a computer-readable recording medium.

Further, any, all, or some of the computing devices in patient monitor 102 may be adapted to execute any operating system, including Linux®, UNIX®, Windows Server®, etc., as well as virtual machines adapted to virtualize execution of a particular operating system, including customized and proprietary operating systems. Patient monitor 102 may be further equipped with components to facilitate communication with other computing devices over one or more network connections, which may include connections to local and wide area networks, wireless and wired networks, public and private networks, and any other communication network enabling communication in the system.

As shown in FIG. 1, patient monitor 102 may be, for example, a patient monitor implemented to monitor various physiological parameters of patient 106 via sensors 104. Patient monitor 102 may include a sensor interface 108, one or more processors 110, a display/graphical user interface ("GUI") 112, a communications interface 114, a memory 116, and a power source (or power connection) 118, all communicating over an internal bus 119. The sensor interface 108 may be implemented in hardware or combination of hardware and software and is used to connect via wired and/or wireless connections to the sensors 104 for gathering physiological data from the patient 106. As noted, the sensors 104 in the present example are ECG electrodes affixed to the skin of the patient 106. A plurality of conductive leads 120, comprising a plurality of conductive cables, are provided for coupling the sensors 104 to the sensor interface 108. In one or more examples, the conductive leads 120 comprise a plurality of ECG cables.

The data signals from the sensors 104 may include, for example, sensor data related to an ECG. The one or more processors 110 may be used for controlling the general operations of the patient monitor 102, as well as processing sensor data received by sensor interface 108. The one or more processors 110 may be any suitable processor-based resource. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of patient monitor 102. In some embodiments, the one or more processors 110 may comprise a processor chipset including, for example and without limitation, one or more co-processors.

The display/GUI 112 may be configured to display various patient data, sensor data, and hospital or patient care information, and includes a user interface implemented for allowing interaction and communication between a user and patient monitor 102. The display/GUI 112 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 112 may provide a means for inputting instructions or information directly to the patient monitor 102. The patient information displayed may, for example, relate to the measured physiological parameters of the patient 106 (e.g., ECG readings).

The communications interface 114 may permit the patient monitor 102 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 114 may include various network cards, interfaces, communication channels, cloud, antennas, and/or circuitry to permit wired and wireless communications with such computing networks and devices. The communications interface 114 may be used to implement, for example, a BLUETOOTH® connection, a cellular network connection, and/or a WIFI® connection with such computing networks and devices. Example wireless communication connections implemented using the communication interface 114 include wireless connections that operate in accordance with, but are not limited to, IEEE802.11 protocol, a Radio Frequency For Consumer Electronics ("RF4CE") protocol, and/or IEEE802.15.4 protocol (e.g., ZigBee® protocol). In essence, any wireless communication protocol may be used.

Additionally, the communications interface 114 may permit direct (i.e., device-to-device) communications (e.g., messaging, signal exchange, etc.) such as from a monitor mount to patient monitor 102 using, for example, a universal serial bus ("USB") connection or other communication protocol interface. The communication interface 114 may also permit direct device-to-device connection to other devices such as to a tablet, computer, or similar electronic device; or to an external storage device or memory.

The memory 116 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 116 may be on-chip or off-chip depending on the implementation of the one or more processors 110. The memory 116 may be used to store any type of instructions and patient data associated with algorithms, processes, or operations for controlling the general functions and operations of the patient monitor 102.

The power source 118 may include a self-contained power source such as a battery pack and/or include an interface to be powered through an electrical outlet, either directly or by way of a monitor mount. The power source 118 may also be a rechargeable battery that can be detached allowing for replacement. In the case of a rechargeable battery, a small built-in back-up battery (or super capacitor) can be provided for continuous power to be provided to the patient monitor 102 during battery replacement. Communication between the components of the patient monitor 102 in this example (may be established using the internal bus 119.

The patient monitor 102 may be attached to one or more of several different types of sensors 104 and may be configured to measure and readout physiological data related to patient 106. As noted, the sensors 104 may be attached to the patient monitor 102 by the conductive leads 120 which may be, for example, cables coupled to sensor interface 108. Additionally, or alternatively, one or more sensors 104 may be connected to sensor interface 108 via a wireless connection. In which case sensor interface 108 may include circuity for receiving data from and sending data to one or more devices using, for example, a WIFI® connection, a cellular network connection, and/or a BLUETOOTH® connection.

The data signals received from the sensors 104 may be analog signals. For example, the data signals for the ECG may be input to the sensor interface 108, which can include an ECG data acquisition circuit (not shown separately in FIG. 1). An ECG data acquisition circuit may include amplifying and filtering circuity as well as analog-to-digital (A/D) circuity that converts the analog signal to a digital signal using amplification, filtering, and A/D conversion methods. In the event that the ECG sensor is a wireless sensor, the sensor interface 108 may receive the data signals from a wireless communication module (not shown in FIG. 1). Thus, the sensor interface 108 is a component which may be configured to interface with the one or more sensors 104 and receive sensor data therefrom.

As further described herein, the processing performed by an ECG data acquisition circuit may generate analog data waveforms or digital data waveforms that are analyzed by, in this particular embodiment, a microcontroller. However, other embodiments may use other kinds of processors disclosed above. The microcontroller may be one of the processors 110.

The one or more processors 110, for example, may analyze the ECG waveforms to identify certain waveform characteristics and threshold levels indicative of conditions (abnormal and normal) of the patient 106 using one or more monitoring methods. A monitoring method may include comparing an analog or a digital waveform characteristic or an analog or digital value to one or more threshold values and generating a comparison result based thereon. The microcontroller may be, for example, a processor, an FPGA, an ASIC, a DSP, a microcontroller, or similar processing device.

The microcontroller may include a memory (an on-chip memory) or use a separate memory 116 (an off-chip memory). The memory may be, for example, a RAM, a memory buffer, a hard drive, a database, an EPROM, an EEPROM, a ROM, a flash memory, a hard disk, or any other non-transitory computer readable medium. The memory 116 may store software or algorithms with executable instructions and the microcontroller may execute a set of instructions of the software or algorithms in association with executing different operations and functions of the patient monitor 102 such as analyzing the digital data waveforms related to the data signals from the sensors 104.

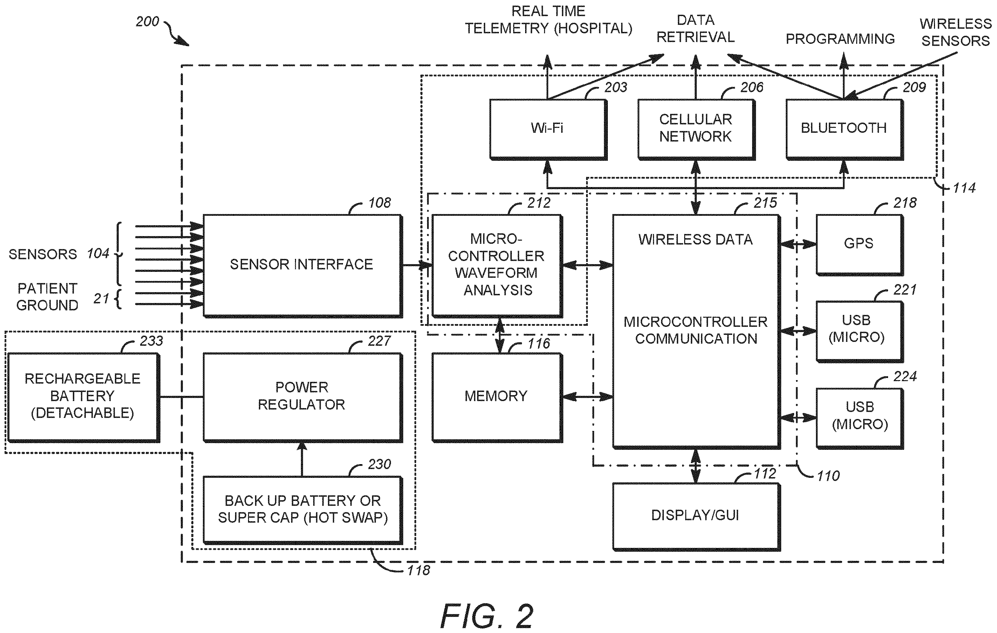

To further an understanding of this particular aspect of the claimed subject matter, FIG. 2 is a schematic diagram of one particular example of a physiological monitoring device 200 such as the physiological monitoring device 100 in FIG. 1. The physiological monitoring device 200 is, in this particular embodiment, attached to several different types of the sensors 104 (including electrodes or other similar devices) known in the art for gathering physiological data related to the patient 106 (e.g., as shown on the left side of FIG. 1). The sensors 104 are communicatively coupled to physiological monitoring device 200 by, for example, a wired connection input to the sensor interface 108.

It is contemplated by the present disclosure that the physiological monitoring device 200 can also be connected to other wireless sensors (not shown) using the communication interface 114 of FIG. 1. The communication interface 114 in these embodiments includes circuity for receiving data from and sending data to one or more devices using, for example, a BLUETOOTH® connection 209. The communications interface 114 shown in FIG. 1 is represented in FIG. 2 by the combination of microcontroller 212 and elements 203-209.

The data signals from the sensors 104 received by the physiological monitoring device 200 include data related to, for example, an ECG, SpO2, NIBP, temperature, and/or etCO2. The data signals received from an ECG sensor and the SpO2 sensor can be analog signals. The data signals for the ECG and the SpO2 are input to the sensor interface 108, which can include an ECG data acquisition circuit and a SpO2 data acquisition circuit. Both the ECG data acquisition circuit and the SpO2 data acquisition circuit include amplifying and filtering circuity as well as analog-to-digital (A/D) circuity that convert the analog signal to a digital signal using amplification, filtering, and A/D conversion methods known in the art.

As another example, the data signals related to NIBP, temperature, and etCO2 can be received from the sensors 104 to the sensor interface 108, which can include a physiological parameter interface such as serial interface circuitry for receiving and processing the data signals related to NIBP, temperature, and etCO2. The ECG data acquisition circuit, an SpO2 data acquisition circuit, and a physiological parameter interface are described as part of the sensor interface 108. However, it is contemplated by the present disclosure that the ECG data acquisition circuit, the SpO2 data acquisition circuit, and the physiological parameter interface can be implemented as circuits separate from the sensor interface 108.

The processing performed by the ECG data acquisition circuit, the SpO2 data acquisition circuit, and external physiological parameter interface produces digital data waveforms that are analyzed by the microcontroller 212. The processors 3 shown in FIG. 1 are represented in FIG. 2 as microcontrollers 212 and 215. The microcontroller 212, for example, analyzes the digital waveforms to identify certain digital waveform characteristics and threshold levels indicative of conditions (abnormal and normal) of the patient 106 using methods known in the art. The microcontroller 212 includes a memory or uses the memory 116.

The memory stores software or algorithms with executable instructions and the microcontroller 212 can execute a set of instructions of the software or algorithms in association with executing different operations and functions of the physiological monitoring device 200 such as analyzing the digital data waveforms related to the data signals from the sensors 104. The results of the operations performed by the microcontroller 212 are passed to the microcontroller 215. The microcontroller 215 includes a memory or uses the memory 116.

As noted above, in FIG. 2, the communication interface 114 shown in FIG. 1 is represented by the combination of microcontroller 215 and elements 203-224. For example, the microcontroller 215 includes communication interface circuitry for establishing communication connections with various devices and networks using both wired and wireless connections, and transmitting physiological data, patient and transport information (e.g., transport times and patient location information), results of the analysis by the microcontroller 212, and alerts and/or alarms to the patient 106, clinicians and/or caregivers. The memory 116 stores software or algorithms with executable instructions and the microcontroller 215 can execute a set of instructions of the software or algorithms in association with establishing the communication connections.

As shown in FIG. 2, wireless communication connections established by the communication interface circuity of microcontroller 215 include a BLUETOOTH® connection 209, a cellular network connection 206, and a WIFI® connection 203. The wireless communication connections can allow, for example, patient and hospital information, alerts, and physiological data to be transmitted in real-time within a hospital wireless communications network (e.g., WIFI®) as well as allow for patient and hospital information, alerts, and physiological data to be transmitted in real-time to other devices (e.g., BLUETOOTH® 209 and/or cellular networks 206).

It is also contemplated by the present disclosure that the communication connections established by the microcontroller 215 permit communications over other types of wireless networks using alternate hospital wireless communications such as wireless medical telemetry service ("WMTS"), which can operate at specified frequencies (e.g., 1.4 GHz). Other wireless communication connections can include wireless connections that operate in accordance with, but are not limited to, IEEE802.11 protocol, a Radio Frequency For Consumer Electronics (RF4CE) protocol, ZigBee protocol, and/or IEEE802.15.4 protocol.

The BLUETOOTH® connection 209 can also be used to provide the transfer of data to a nearby device (e.g., tablet) for review of data and/or changing of operational settings of the physiological monitoring device 200. The microcontroller 215 of the physiological monitoring device 200 provides a communication connection by direct wired (e.g., hard-wired) connections for transferring data using, for example, a USB connection 221 to a tablet, PC, or similar electronic device (not shown); or using, for example, a USB connection 224 to an external storage device or memory. Additionally, the microcontroller 215 includes a connection to a display/GUI 112 including a GUI for displaying patient information, physiological data or measured data, measurement schedules, alerts or alarms for the patient, clinicians and/or caregiver's information. Although the physiological monitoring device 200 is described in FIG. 1 as having two microcontrollers 212 and 215, it is contemplated by the disclosure of the present application that one microcontroller can be implemented to perform the functions of the two microcontrollers 212 and 215.

As shown in FIG. 2, the physiological monitoring device 200 includes a global positioning system (GPS) or other location data system 218 that can be connected to the communication interface circuity of microcontroller 215 so that the physiological monitoring device can transmit to the clinician, caregiver, or other devices the location of the patient 106 at all times including the location of the patient 106. Additionally, the location of the patient 106 can be used by the microcontroller 215 to determine an estimated time of arrival of the patient 106.

For example, location data provided by the location data system 218, which may include information on a floor level, can be compared to stored information related to a hospital layout or a hospital map as well as information related to a patient's scheduled care (e.g., treatment or procedure scheduled for the patient 106 in a patient care area within the hospital). Based on the comparison results, the microcontroller 215 can determine the estimated time of arrival of the patient 106 to the patient care area within the hospital. The estimated time of arrival can be transmitted by the communication interface circuity of microcontroller 215 to, for example, the hospital wireless communications system.

Additionally, if it is determined by the microcontroller 215 that the patient 106 is not within the vicinity of the hospital wireless communications system (e.g., based on input from the location data system 218), the pertinent physiological data can be recorded and stored in the memory 116. Additionally, if the BLUETOOTH® connection 209 or WIFI® connection 203 are not available (e.g., out of transmission range or not operable), then the microcontroller can store the physiological data in the memory 116 for later transmission when the BLUETOOTH® connection or WIFI® connection becomes available.

The power source 118 shown in FIG. 1 is represented by elements 227-233 in FIG. 2. As shown in FIG. 2, the power can be supplied using a rechargeable battery 233 that can be detached allowing for replacement. The rechargeable battery 233 may be, for example, a rechargeable lithium-ion battery. Additionally, a small built-in backup battery 230 (or supercapacitor) is provided for continuous power to the physiological monitoring device 200 during battery replacement. A power regulator or regulation circuit 227 is provided between the rechargeable battery 233 and small backup battery 230 to control which battery provides power to the physiological monitoring device 200.

The physiological monitoring device 200 also includes a patient ground connection 236. The patient ground connection 236 can be used as a ground for single ended unipolar input amplifiers (e.g., precordial leads), or as a ground for bipolar input amplifiers (e.g., limb leads). It is also contemplated by the present disclosure that the power regulator 227 can include a self-contained power source such as a battery pack and/or include an interface to be powered through an electrical outlet (either directly or by way of a monitor mount). Communication between the components of the physiological monitoring device 200 can be established using an internal bus similar to the internal bus 119 discussed with reference to FIG. 1.

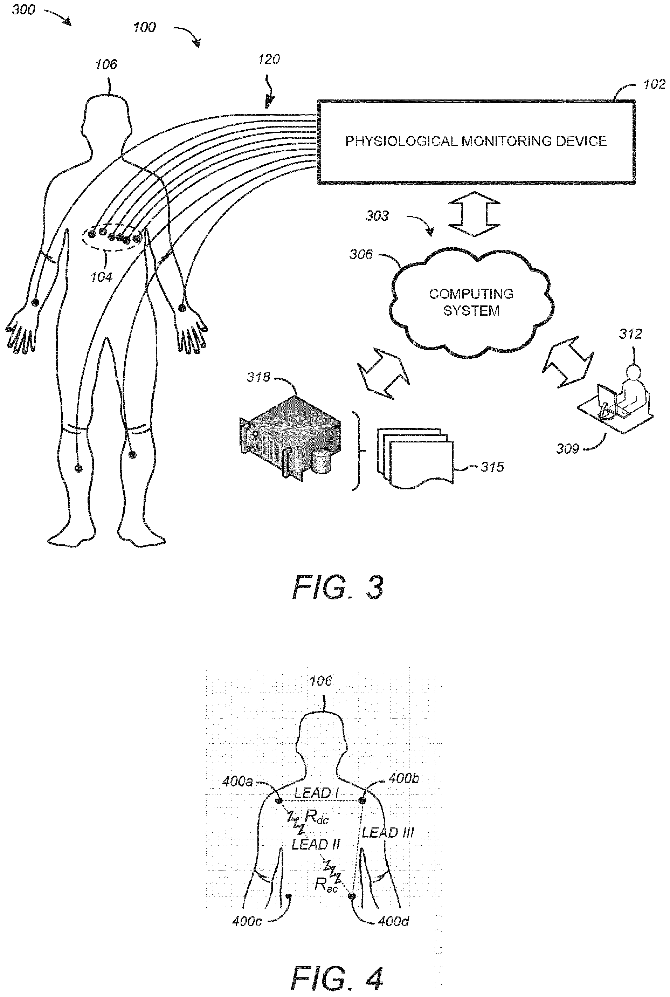

The physiological monitoring system 100 may also be deployed within the context of a larger patient monitoring system 300, shown in FIG. 3. FIG. 3 is a schematic diagram of an example of a patient monitoring system 300 including a computing system 303 and the physiological monitoring system 100 of FIG. 1 according to an embodiment of the present disclosure. The computing system 303 may be a distributed computing environment including a network or cloud 306 over which the physiological monitoring system 100 pushes and/or pulls data and information. The communications among the physiological monitoring device 102, the computing system 306, and other resources of the patient monitoring system 300 may be either wireless or wired depending upon the technical capabilities of the various components.

For example, the facility (not otherwise shown) in which the patient 106 is located may include a central monitoring station 309 from which a caregiver 312 may monitor the physical condition of multiple patients 106 concurrently. The physiological monitoring device 102, using, for example, the communications interface 114 shown in FIG. 1, may push sensed physiological parameters and/or signals representative thereof to the central monitoring station 309 over the computing system 306. The pushed data and information may then be displayed for the caregiver 312 for review and consideration.

For another example, the physiological monitoring device 100 may pull information from an electronic medical record 315 for the patient 106 from a records repository 318 using, for example, the communications interface 114 shown in FIG. 1. The pulled information can then be displayed by the physiological monitoring device 102. Such pulled information might include, depending on the implementation and without limitation, medication regimens, diagnosed medical conditions, medical history, historical medical data, etc. Similarly, data sensed by or entered at the physiological monitoring device 102 may be pushed to, for instance, the ERM 315 for storage and later retrieval.

FIG. 4 presents a patient model illustrating these measurement and acquisition of the impedance respiration signals in the embodiments disclosed herein. Note that alternative embodiments may use different patient models than the one shown in FIG. 4. Note also that the placement of the four sensors 400a-400d using three leads is determined so as to measure the thoracic resistance through the chest of the patient 106. $R_{dc}$ is the constant thoracic impedance while $R_{ac}$ is the measured change to impedance due to breathing. More particularly, in order to measure the change in impedance caused by breathing, impedance respiration measurement uses the ECG electrodes that inject a weak, high frequency square wave current (~70 nA, 40 kHz) into the patient 106 using one of three lead selections, namely Lead I, Lead II, or Lead III. The voltage across the sensors 400a-400d is measured from which the impedance signal is derived. The impedance signal then undergoes hardware and software filtering, as described below.

Those in the art having the benefit of this disclosure will appreciate that lead selection will depend, at least to some degree, on the physiological parameter being determined. This is because the ECG signal should reflect that physiological parameter as accurately or strongly as possible from among the available ECG signals. Hence, in the illustrated embodiments, the leads indicated in FIG. 4 are preferred since it is the impedance respiration that is determined. The lead to one of the patient's legs would be much less preferred.

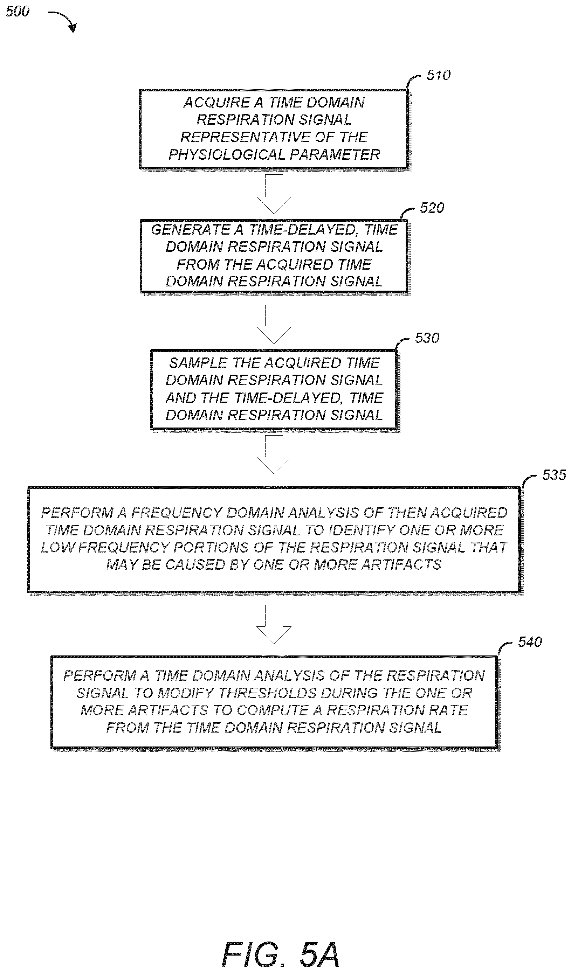

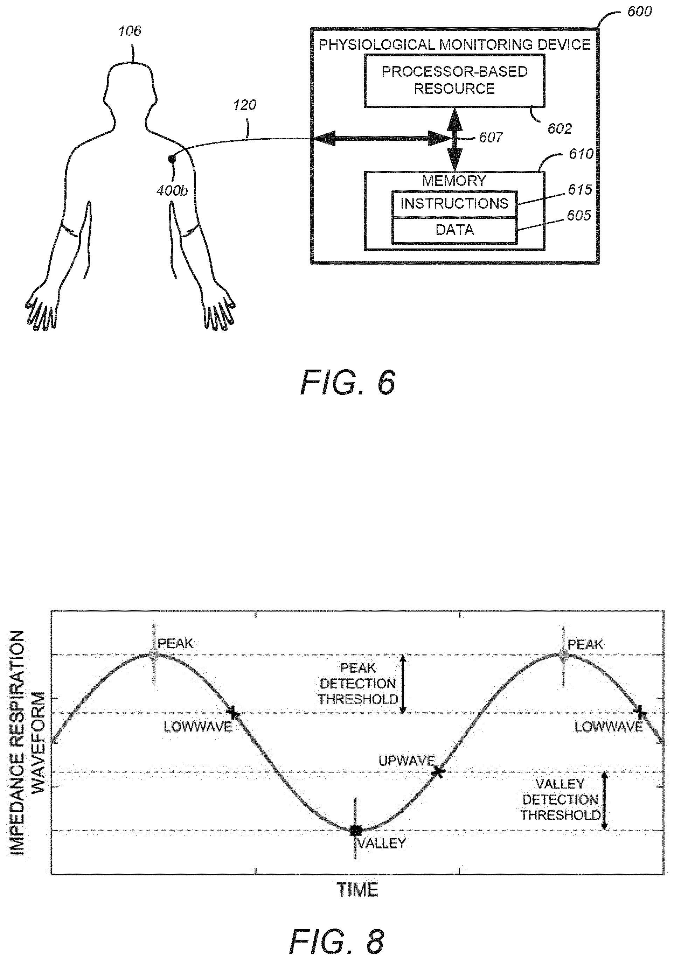

FIG. 5A-FIG. 5B illustrate a method 500 for use in quantifying a physiological parameter of a monitored patient in accordance with one or more embodiments. FIG. 6 schematically depicts one particular implementation of a physiological monitoring device 600. The depiction of the physiological monitoring device 600 is simplified from what is shown in FIG. 1 and FIG. 2 so as not to obscure those aspects of the claimed subject matter now under discussion. More particularly, as illustrated in FIG. 6, a processor-based resource 602 communicates with a memory 610 over an internal bus system 607. Thus, some of the details discussed relative to and shown in FIG. 1 and FIG. 2 have been omitted for purposes of this discussion although such details will be pertinent in most embodiments.

The processor-based resource 602 may be, for example, one of the one or more processors 110 shown in FIG. 1, including one or both of the microcontrollers 212, 215 shown in FIG. 2. The processor-based resource 602 operates under programmed control of the instructions 615. The processor-based resource 602 therefore communicates with the memory 610 over the bus system 607 to, among other things, execute the instructions 615 stored thereon.

The execution of the instructions may cause the processor-based resource 602 to perform certain tasks such as some portions of the method 500 outlined in FIG. 5A-FIG. 5B. However, depending on the environment, the processor-based resource 602 may also perform other tasks such as pushing data to other computing resources, transmitting alarms, *etc.* Thus, in this sense, the processor-based resources 602, as well as the physiological monitoring device 600 as a whole, may be considered to be "programmed" or "configured" to perform certain functions as described herein.

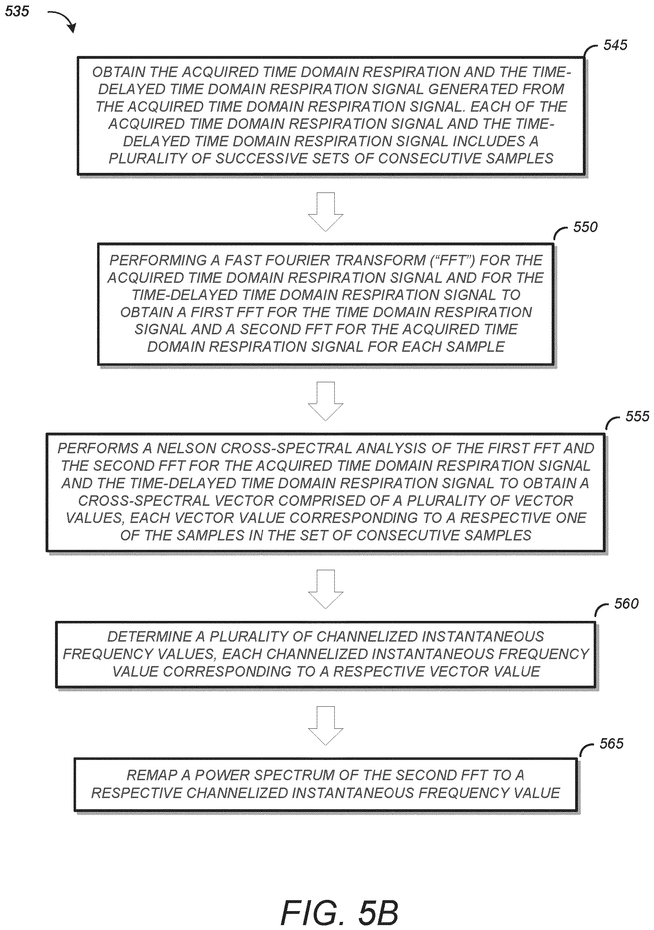

Referring now collectively to FIG. 5B and FIG. 6, the method 500 begins by acquiring (at 510) a time domain respiration signal representative of the physiological parameter. This time domain respiration signal is acquired via the selected lead 120 and ECG sensor 400b as described above relative to FIG. 4. Note that the ECG sensor 400b is representative and that other ECG sensors (not shown in FIG. 6) may be used in other embodiments. The acquired time domain respiration signal is transmitted from the ECG sensor 400b to the physiological monitoring device 600 over the lead 120.

The acquired time domain respiration signal as received by the physiological monitoring device may be in either analog or digital form. If received in analog form, it is converted to digital and stored as data 605 in the memory 610. The memory 610 in this embodiment is off-chip and includes both RAM and ROM in both volatile and non-volatile memory. The data 605 may be stored in any suitable kind of data structure.

Note that the information derived from the acquired time domain respiration signal will typically be displayed in real time or near real time in most embodiments. Thus, the data 605 representing the acquired time domain respiration signal may be temporarily stored, or buffered, in the memory 610. Some embodiments may, however, store the data 605 representing the acquired time domain respiration signal in portions of memory 610 more suitable for long term storage in addition to buffering. Some embodiments may even push such data 605 to other computing resources for longer term storage as discussed relative to FIG. 3.

The method 500 then continues (at 520) by generating a time-delayed, time domain respiration signal from the acquired time domain respiration signal. This may be performed either in hardware (not shown) or in software by the processor-based resource 602. The illustrated embodiment performs this task in software and stores the time-delayed, time domain respiration signal as a separate set of data 605 in the memory 610. Specific factors such as the amount of the delay are implementation specific and illustrative examples are provided below. The acquired time domain respiration signal and the time-delayed, time domain respiration signal are then sampled (at 530).

Once the time domain respiration signal and the time-delayed time domain respiration signal are obtained and sampled, a frequency domain analysis of the acquired time domain respiration signal is performed to identify one or more low frequency portions of the respiration signal that may be caused by one or more artifacts. The method 500 then performs a time domain analysis of the respiration signal to modify thresholds during the one or more artifacts to compute a respiration rate from the time domain respiration signal.

The frequency domain analysis (at 535) in one particular embodiment is illustrated in FIG. 5B. The frequency domain analysis (at 535) begins by obtaining (at 545) the acquired time domain respiration and the time-delayed time domain respiration signal generated from the acquired time domain respiration signal. Each of the acquired time domain respiration signal and the time-delayed time domain respiration signal includes a plurality of successive sets of consecutive samples. "Obtaining" in this context may include retrieving from memory or acquiring and processing. The frequency domain analysis (at 535) continues by performing (at 550) a Fast Fourier Transform ("FFT") for the acquired time domain respiration signal and for the time-delayed time domain respiration signal to obtain a first FFT for the time domain respiration signal and a second FFT for the acquired time domain respiration signal for each sample.

The frequency domain analysis (at 535), in this particular embodiment, then performs (at 550) a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples. Next, a plurality of channelized instantaneous frequency values is determined (at 560), each channelized instantaneous frequency value corresponding to a respective vector value. Then, a power spectrum for the second FFT is remapped (at 565) to a respective channelized instantaneous frequency value.

The frequency domain signal representative of a physiological parameter can then be used to, for example, assist in removing artifacts. In one embodiment, the technique described above is used to remove one or more kinds of artifacts from an acquired ECG signal from which a patient's impedance respiration is obtained. However, as noted above, the disclosed technique is not limited to use in this context and may be used in the measurement and monitoring of any physiological parameter that may be reflected in an ECG signal.

As discussed above, one kind of artifact encountered in impedance resistance measurement are known as "movement artifacts", or "motion artifacts". These will be referred to as "motion artifacts" hereafter. Artifacts in the impedance respiration signal due to movement are typically signals of lower frequency. A frequency-based Nelson Cross Spectral method analysis assists in detecting these movement artifacts. In this embodiment, a Nelson Cross-spectral analysis is applied to the Fourier transform of the acquired respiration signal-the acquired time domain respiration signal representative of the physiological parameter-and a time-delayed signal generated therefrom. The spectral analysis provides the maximum estimate of power and the frequency at which the maximum power was obtained. The power estimates at frequency information are used to help discriminate movement artifacts from real breaths in the impedance respiration signal and modify/adapt a breath detection threshold.

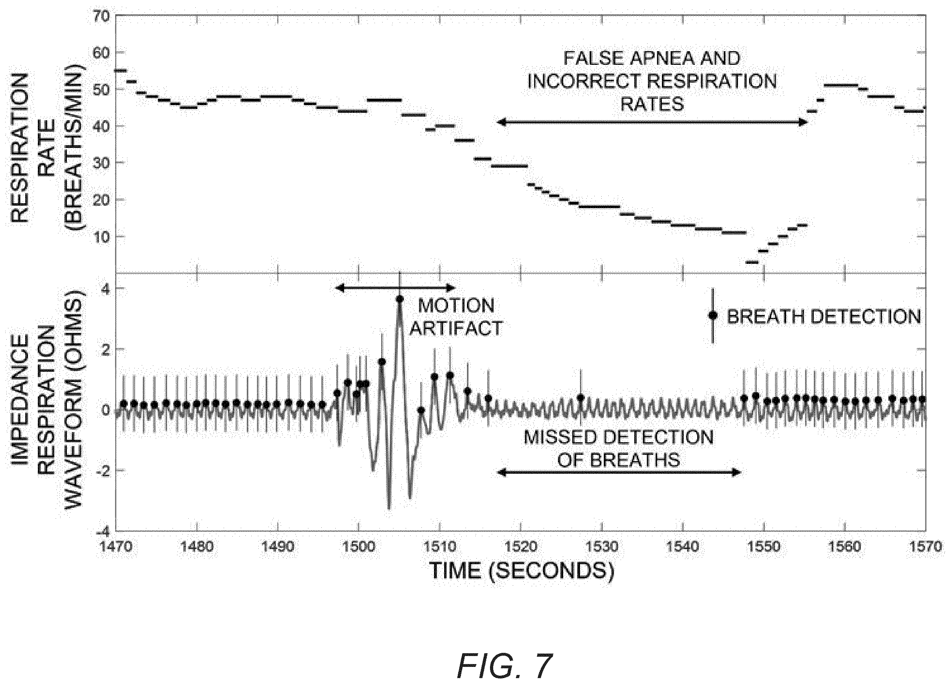

Of particular concern is movement (both spontaneous and external) that could cause abrupt and high amplitude variations in the amplitude of impedance respiration waveforms. These high amplitude data points could be erroneously detected as breaths by the monitoring technique as shown in FIG. 7. FIG. 7 is an example of movement artifacts on the impedance respiration waveform and the false apnea alarms due to undetected breaths. This problem can be resolved in two steps: First, by detecting instances of data which have high amplitude artifacts either due to movement or other physiological anomalies. The second step involves adjusting the detection breath threshold during these events.

Turning first to the particulars of the frequency analysis, in this particular embodiment, the Nelson Cross Spectral analysis uses the last 300 samples, typically taken over six seconds, of respiration data extracted from the ECG signal. The frequency analysis is based on Nelson's cross-spectral methods with reassignment as discussed in Nelson, D., "Cross-Spectral Methods for Processing Speech", 110 J. Acoust. Soc. Am. 2575 (2001). The reassignment process in this cross-spectral method remaps the sample points of the Fourier spectrum to channelized instantaneous frequencies ("CIF") by concentrating the smeared-out points around tighter lines, effectively "sharpening" it as discussed in Fulop, S., "An Accurate Means for Measuring Formants", Dept. of Linguistics, Dept. of Computer Science, The University of Chicago (2003).

This cross-spectral method is performed using the acquired time domain respiration signal that is representative of the physiological parameter-impedance resistance in this example-and a time-delayed copy thereof. The time delay should be as small as possible. In one implementation, the time delay is one sample. The chosen length of the acquired time domain respiration signal was empirically determined at 300 samples, about six seconds, acquired at a rate of 50 samples per second.

The frequency analysis begins by performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample. A Hanning window is then applied to the FFT of each of the 300 sample, six second segments. The Hanning window is applied to reduce artifacts introduced by performing the discrete Fourier transform.

All discrete Fourier transforms in this embodiment are fast Fourier transforms ("FFT") Algorithm. The FFT length, or desired frame length for the Fourier transforms that will be performed, was chosen as the power of 2 of four times the length of the signal, or $2^{nextpow2(4*301)} = 2048$. By choosing a frame much longer than the signal (zero padded), the Fourier spectral magnitudes themselves are not changed much, but there will be a finer quantization of the frequency scale than when using the unpadded signal.

Next, the Nelson's cross-spectral vector is computed as a dot product of the FFT of the two signals. The cross-spectral vector is a vector of complex numbers whose phase angles encode the information about the instantaneous frequency of the signal in each frequency channel. The channelized instantaneous frequency ("CIF") vector is computed from the argument (i.e., the phase angles) of the cross-spectrum. This is vector of real numbers which give the CIF (in Hz) of each discrete frequency in the discrete Fourier spectrum.

In the illustrated embodiment, this CIF is then wrapped in $2\pi$ since it would consist of radian angles between 0 and $2\pi$. Next, the magnitude power spectrum ("PS") of the signal frame is computed. The reassigned Nelson spectrum plot is obtained by assigning any CIF values outside the frequency range of 0-25Hz to a magnitude of 0. (Note that, because the samples were acquired at a rate of 50 samples per second, the FFT maximum frequency range is therefore 50/2=25Hz.) These values will appear benignly at the origin of the generated Nelson spectrum.

Now that the frequency analysis has been described, the events to be monitored for will be discussed. The monitoring for these events occurs in the time domain. Turning now to breath detection, the disclosed embodiment performs a peak and valley detection on the respiration signal. FIG. 8 illustrates some of the concepts associated with peak and valley detection that will be used in this discussion. The peak-detection is based on an assumption that a valid breath consists of a valid peak followed by a valid valley.

The technique is initialized by looking for a local peak. This is performed by comparing the amplitude of the incoming respiration data point to a set peak value. If the new data sample value is greater than the set peak value, it becomes the new set peak value. When a peak has been found, it is declared as a valid peak after the signal amplitude falls below the breath detection threshold labelled as the 'lowwave' point.

Then the search for a valley begins. To maintain a minimum detectable breath of $0.2\,\Omega$, the initial amplitude for the search of the next valley is $0.2\,\Omega$ below the amplitude of the last valid peak. The next valley would have to occur below this point. The technique then looks for a local minimum. This is performed by comparing the amplitude of the incoming respiration data point to the previous valley value. If the new data sample value is lower than the previous valley value, it becomes the new valley. When a valley has been found, it will be declared as a valid valley after the signal amplitude increases above the breath detection threshold level labelled as the 'upwave' point. Then, the search for a peak starts all over again, where the initial amplitude for the search of the next peak is $0.2\,\Omega$ above the amplitude of the last valid valley.

Some embodiments employ an adaptive threshold determination for the breath detection threshold. Turning now to adaptive threshold determination, in this particular embodiment, the lowwave and upwave levels are based on adaptive threshold values computed using the peak to its previous valley's amplitude of the last 8 detected breaths. It is computed whenever a new valid peak (breath) is detected, as one third of the average of four peak-to-valley amplitude values, where the two greatest and two smallest values of eight amplitude values are dropped. This has been referred to as four-out-of-eight average previously.

$$Threshold = \frac{1}{3}\frac{1}{4}\sum_{3}^{6} sort(Peak\_to\_Valley\_Amplitude) \tag{1}$$

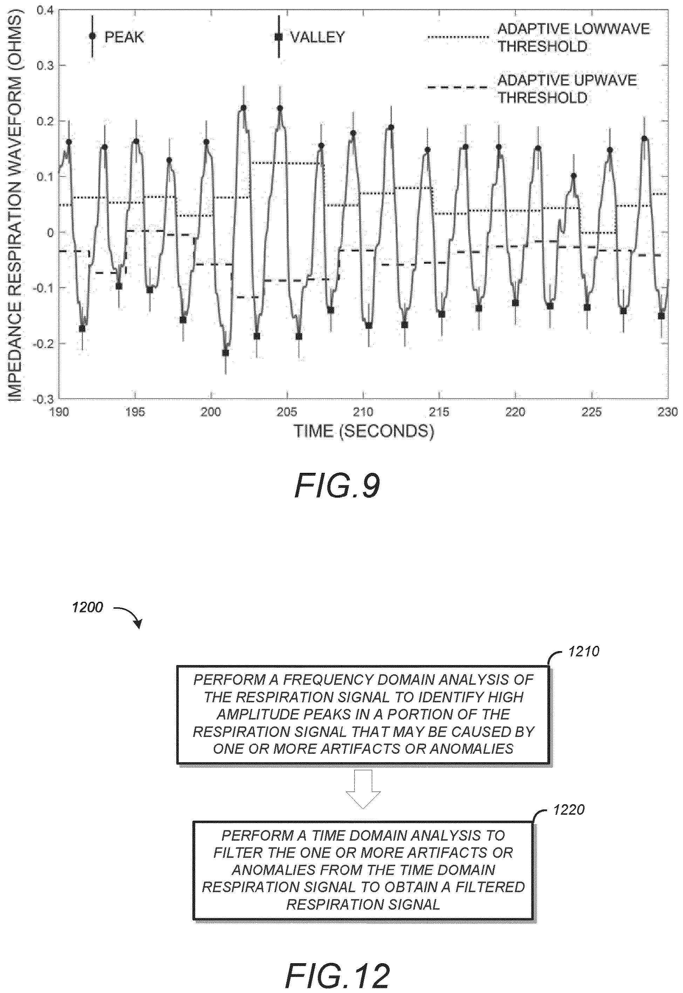

Upon the detection of a local peak, the lowwave is set as the peak amplitude minus the computed adaptive threshold as illustrated in FIG. 9. A local peak is assigned as a valid breath when the respiratory signal value falls below the lowwave threshold. Similarly, upon the detection of a valley, the upwave is set as the valley amplitude plus the computed adaptive threshold. Note that, in this particular embodiment, adaptive threshold determination is not available until eight breaths are detected. The adaptive threshold is further modified during cardiac artifacts, movement artifacts and shallow breaths and will be discussed in their respective subsections.

Whenever a valid breath is found, the timestamp of the breath (local maximum) is saved in terms of samples elapsed since respiration monitoring was enabled. The timestamp is saved in a variable called *PeakTime* of size nine and the variable *PeakTime* saves the locations of the last nine detected breaths. When any new breath is detected all the breath locations in the variable *PeakTime* are shifted left once and the new breath location is saved at the last index of the variable *PeakTime*. This timestamp is used in the computation of breath-to-breath intervals to estimate the respiration rate. The timestamp is also an output of the technique that is displayed on the display of the physiological monitoring device and, in some embodiments, the central monitoring station. The timestamp is displayed as a vertical line to indicate where breaths were found.

Once a valid peak is found, the time elapsed from its previous valid peak (in samples) is also stored in an array. When the array contains eight consecutive time intervals, the respiration rate ("RR") in breaths/min is computed as four-out-of-eight average of that array as below,

$$RR = \frac{60 * 50}{\frac{1}{4}\sum_{3}^{6} sort(C\_tstamp)} \tag{2}$$

At the beginning of respiration monitoring when there are an insufficient number of breath-breath intervals to compute the respiration rate using Eqn. (3), an alternative formula between breaths 2 and 9 is used as below,

$$RR = \frac{60 * 50}{mean(C\_tstamp)} \tag{3}$$

Since the technique is invoked every pollcount (300 msec), the respiration rate is held until a new valid peak (breath) is detected. However, if no new breath is detected for 5 s, the respiration rate is decayed based on the time interval between the current sample time and the eight to the last breath as below,

$$RR = \frac{60 * 50}{\frac{1}{8}(Current\_Sample\_Count - PeakTime(2))} \tag{4}$$

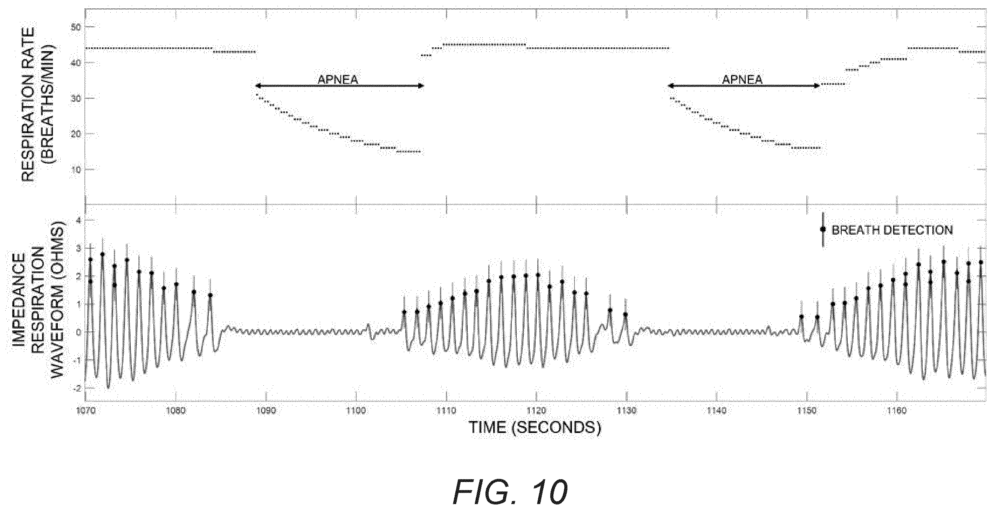

where, *PeakTime(2)* is the 2nd index of the variable *PeakTime* (of size 9 as described above). It indicates the eight to the last detected breath. If an apnea state is not reached, the technique reverts to using the normal RR formula in Eqn. (2) as soon as a new breath is detected. If an apneic state is encountered, the physiological state of the subject could have possibly changed. Therefore, at the end of an apnea, the respiration rate computation should not include breath-breath intervals before the apnea. The technique holds the computed decayed respiration rate between the end of the apnea and the 2nd breath. It then uses Eqn. (4) for computation of respiration rate between breaths 2 and 9 after the end of the apnea. After the 9th breath is detected, it reverts back to Eqn. (2). FIG. 10 shows the computed respiration rate and its decay during a pause in breathing of duration greater than 5s.

An apnea state is generated when there are no breaths detected for a time interval determined by the user (apnea delay time = 15 seconds by default). The time elapsed since the last peak is counted from the actual location of the peak and not from the delayed location that the peak was validated as a breath. Note that 'no breaths detected' indicates that a valid peak or a valid valley was not found based on the detection threshold. An apnea state cannot be generated when there are an insufficient number of detected breaths (e.g., eight, in this embodiment). FIG. 10 also shows the detection of an apnea with apnea delay time set at 10s.

Now that the events to be monitored for and the adaptive detection threshold have been defined, the detection and filtering will now be discussed. As described above, movement (both spontaneous and external) could cause abrupt and high amplitude variations in the impedance respiration waveforms. These high amplitudes could be erroneously detected as breaths. Where the adaptive detection threshold is used, and since the adaptive detection threshold is based on breath amplitudes, the large amplitudes during movements could increase the detection threshold which could lead to real breaths after the end of movement go undetected causing false apnea alarms, as illustrated in FIG. 7. This issue can be solved in two steps. First, by detecting instances of data which could have high amplitudes either due to movement or other physiological anomalies. Second, by clamping the detection threshold during these events.

The first step uses the output of the frequency analysis employing the Nelson cross-spectral analysis described above. The frequency analysis outputs the value of maximum and its corresponding frequency (converted to rate) from the most recent respiration data segment of, in this particular embodiment, duration 6s. If this frequency lies outside of the Respiration Rate range ($\pm$ 10 breaths per min, in this embodiment) and the maximum power is above an empirically determined threshold (based on the power of an average breath amplitude), then an artifact/anomaly condition is said to have occurred.

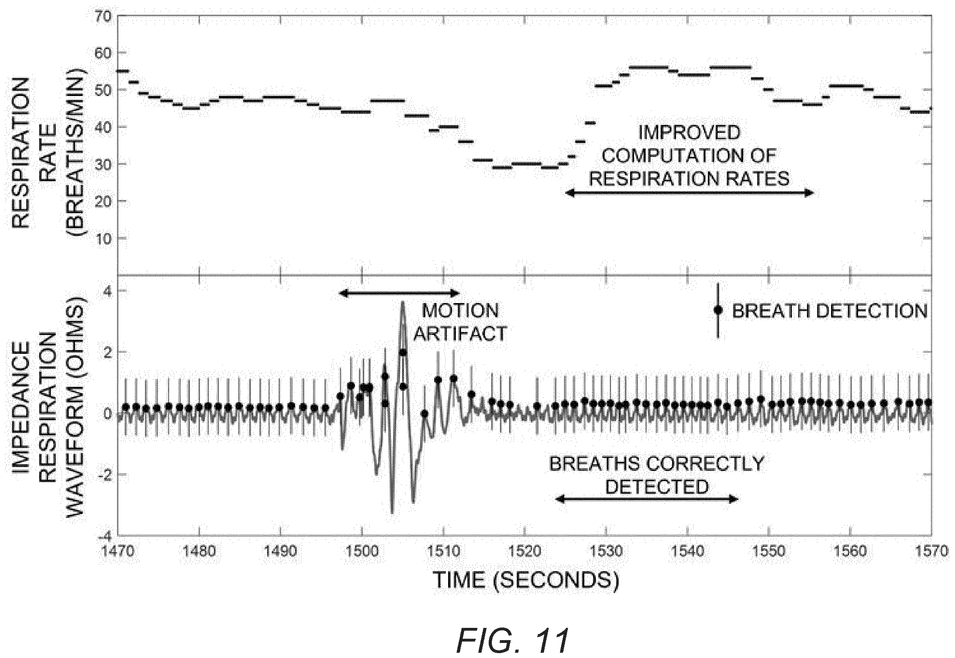

The second step then clamps the value of Peak-to-Valley amplitude at, in this particular embodiment, 2.7 $\Omega$. This value was chosen assuming that the average Peak-to-Valley amplitude is 0.9 $\Omega$, and that any signal with three times this value is due to a movement artifact. The clamps are applied at an equal amplitude from both the peak and valley, while maintaining a new Peak-to-Valley amplitude at 2.7 $\Omega$. The adaptive detection threshold discussed above is then computed using this clamped value. As an additional guard, the breath detection threshold during these events is adjusted such that it cannot go above 0.9 $\Omega$. Limiting the amplitude assures that real breaths around an average breath amplitude after the end of movement can still be detected. The respiration rate obtained after performing this correction is shown in FIG. 11 and could be compared with FIG. 7 where the correction was not applied.

Since the detection of movement artifacts and anomalies in signal are based on frequency domain analysis, certain consistent physiological patterns in the respiration waveform could cause the movement artifact marker to stay high for a long time. To avoid this, the technique has provisions to turn the artifact marker off after 10s.

As mentioned before the frequency domain analysis is performed on the waveform before cardiac artifact filtering. This allows us to identify sections of data where cardiac artifact is dominant. The breath detection threshold during these events is adjusted such that it cannot fall below the default average breath amplitude of 0.3 $\Omega$. The rationale behind this adjustment is that false breaths due to a cardiac artifact are typically less than 0.3 $\Omega$. Setting the threshold higher than 0.3 $\Omega$ ensures that these artifacts are not counted as real breaths, in cases where the cardiac filter is turned off or during ineffective cardiac filtering. This is essentially helpful during a real apneic event when the non-detection of these false breaths can accurately sound an apnea alarm.

As those in the art having the benefit of this disclosure will appreciate, it is possible that multiple events being monitored for occur concurrently, if not simultaneously. Where this occurs, one embodiment employs a priority scheme to order the multiple events for analysis and reporting. The highest priority is assigned to events that contain a dominant cardiac artifact component in the original impedance respiration signal. Here the lower limit of the detection threshold is capped to 0.3 $\Omega$. The next priority is assigned to events with movement artifacts/anomalies. Here the upper limit of the threshold is capped to 0.9 $\Omega$. When none of these events occur, the detection threshold follows the approach described above.

FIG. 12 illustrates a method 1200 in accordance with one or more embodiments as described above. The method 1200 is a method for detecting events in a respiration signal representing the respiration of a monitored patient. The method 1200 begins by performing (at 1210) a frequency domain analysis of the respiration signal to identify high amplitude peaks in a portion of the respiration signal that may be caused by one or more artifacts or anomalies. The method 1200 then continues by performing (at 1220) a time domain analysis to filter the one or more artifacts or anomalies from the time domain respiration signal to obtain a filtered respiration signal.

Those in the art may appreciate still further embodiments given the benefit of the present disclosure. For example, frequency analyses other than the Nelson Cross Spectral analyses may be used. Several Matlab® in-built functions were also investigated, including "fft", "pspectrum", "wsst", "fsst", and "wvd". In summary, the Nelson's method was chosen since the Nelson's method had the lowest root mean square ("rms") error and second fastest execution time. The "fft" function had the best execution time, but it has a poor frequency resolution leading to high errors. Other methods were not chosen due to their high execution time. For the length of data, the current implementation uses 300 samples of data. However, lengths as low as 150 could be sufficient in getting an accurate rate. Data of length 300 sample use two 2048-sample FFT in the Nelson's method. If the input length is reduced to a max of 255 samples, the Nelson's method would require only a 1024-sample FFT. Nevertheless, there may be embodiments in which another frequency domain analysis is desirable.

Accordingly, in a first embodiment a method for use in quantifying a physiological parameter of a monitored patient comprises performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

In a second embodiment, the frequency domain analysis in the method of the first embodiment comprises obtaining the acquired time domain respiration and a time-delayed time domain respiration signal generated from the acquired time domain respiration signal, each of the acquired time domain respiration signal and the time-delayed time domain respiration signal including a plurality of successive sets of consecutive samples. A Fast Fourier Transform ("FFT") is performed for the acquired time domain respiration signal and for the time-delayed time domain respiration signal to obtain a first FFT for the time domain respiration signal and a second FFT for the acquired time domain respiration signal for each sample. A Nelson cross-spectral analysis is performed for the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples; a plurality of channelized instantaneous frequency values is determined, each channelized instantaneous frequency value corresponding to a respective vector value; and each of a power spectrum of the second FFT is remapped to a respective channelized instantaneous frequency value.

In a third embodiment, the method of the first embodiment comprises generating the time-delayed, time domain respiration signal from the acquired time domain respiration signal; and sampling the acquired time domain respiration signal and the time-delayed, time domain respiration signal.

In a fourth embodiment, the method of the first embodiment further comprises generating the time-delayed, time domain respiration signal from the acquired time domain respiration signal and sampling the acquired time domain respiration signal and the time-delayed, time domain respiration signal.

In a fifth embodiment, the method of the fourth embodiment further comprises acquiring the time domain respiration signal representative of the physiological parameter.

In a sixth embodiment, the method of the fifth embodiment further comprises filtering the one or more artifacts from the time domain respiration signal;

In a seventh embodiment, the method of the sixth embodiment further comprises displaying the filtered time domain respiration signal.

In an eighth embodiment, the method of the sixth embodiment further comprises determining that an alarm condition exists; and issuing an alarm.

In a ninth embodiment, a physiological monitoring device comprises a processor; and a memory encoded with instructions that, when executed by the processor, performs a method. The method comprises performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

In a tenth embodiment, in the method performed in the ninth embodiment, obtaining a time domain respiration signal representative of a physiological parameter and a time-delayed time domain respiration signal obtained from the acquired time domain respiration signal, each of the time domain respiration signal and the time-delayed time domain respiration signal including a plurality of successive sets of consecutive samples; performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample; performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples; determining a plurality of channelized instantaneous frequency values, each channelized instantaneous frequency value corresponding to a respective vector value; and remapping a power spectrum of the second FFT to a respective channelized instantaneous frequency value.

In an eleventh embodiment, in the physiological monitoring device of the ninth embodiment, the method further comprises generating the time-delayed, time domain respiration signal from the acquired time domain respiration signal and sampling the acquired time domain respiration signal and the time-delayed, time domain respiration signal.

In a twelfth embodiment, in the physiological monitoring device of the eleventh embodiment, the method further comprises acquiring the time domain respiration signal representative of the physiological parameter.

In a thirteenth embodiment, in the physiological monitoring device of the ninth embodiment, the physiological monitoring device further comprises a display; and the method further comprises: filtering the one or more artifacts from the acquired time domain respiration signal; and displaying the filtered time domain respiration signal.

In a fourteenth embodiment, in the physiological monitoring device of the ninth embodiment, the method further comprises determining that an alarm condition exists; and issuing an alarm.

In a fifteenth embodiment, a patient monitoring system, comprises a plurality of electrocardiogram ("ECG") leads over which a time domain respiration signal representative of a physiological parameter may be obtained; and a physiological monitoring device. The physiological monitoring device comprises a processor; and a memory encoded with instructions that, when executed by the processor, performs a method. The method comprises performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

In a sixteenth embodiment, in the method of the fifteenth embodiment the frequency domain analysis comprises: obtaining a time domain respiration signal representative of a physiological parameter and a time-delayed time domain respiration signal obtained from the acquired time domain respiration signal, each of the time domain respiration signal and the time-delayed time domain respiration signal including a plurality of successive sets of consecutive samples; performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample; performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples; determining a plurality of channelized instantaneous frequency values, each channelized instantaneous frequency value corresponding to a respective vector value; and remapping a power spectrum of the second FFT to a respective channelized instantaneous frequency value.

In a seventeenth embodiment, in the patient monitoring system of the fifteenth embodiment, the method further comprises generating the time-delayed, time domain respiration signal from the acquired time domain respiration signal and sampling the acquired time domain respiration signal and the time-delayed, time domain respiration signal.

In an eighteenth embodiment, in the patient monitoring system of the seventeenth embodiment, the method further comprises acquiring the time domain respiration signal representative of the physiological parameter.

In a nineteenth embodiment, in the patient monitoring system of the eighteenth embodiment, the physiological monitoring device further comprises a display; and the method further comprises: filtering the one or more artifacts from the acquired time domain respiration signal; and displaying the filtered time domain respiration signal.

In a twentieth embodiment, in the patient monitoring system of the fifteenth embodiment, the method further comprises determining that an alarm condition exists; and issuing an alarm.

In a twenty-first embodiment, the patient monitoring system of the fifteenth embodiment further comprises a central monitoring station communicating with the physiological monitoring device.

In an twenty-second embodiment, in the patient monitoring system of the twenty-first embodiment, the central monitoring system receives the filtered time domain respiration signal from the physiological monitoring device; and displays the filtered time domain respiration signal.

In a twenty-third embodiment, a method for use in monitoring the physical condition of a patient is substantially as shown and described.

In a twenty-fourth embodiment, a physiological monitoring device is substantially as shown and described.

In a twenty-fifth embodiment, a patient monitoring system is substantially as shown and described.

The expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1 %, unless otherwise expressly specified. Further, herein the term "substantially" as used herein means a majority, or almost all, or all, or an amount with a range of about 51 % to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the following, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the following description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

ECG signal processing, as used herein, refers to, without limitation, manipulating an analog signal in such a way that the signal meets the requirements of a next stage for further processing. ECG signal processing may include converting between analog and digital realms (e.g., via an analog-to-digital or digital-to-analog converter), amplification, filtering, converting, biasing, range matching, isolation and any other processes required to make a sensor output suitable for processing.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the spirit and scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the spirit and scope of the present disclosure.

Various modifications to the disclosure will therefore be readily apparent to those skilled in the art, and the generic principles defined herein may be applied to other variations without departing from the spirit or scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

This disclosure includes the following numbered Paragraphs or "Paras":

Para 1. A method for use in monitoring the physical condition of a patient, comprising: performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

Para 2. The method of Para 1, wherein the frequency domain analysis comprises: obtaining the acquired time domain respiration and a time-delayed time domain respiration signal generated from the acquired time domain respiration signal, each of the acquired time domain respiration signal and the time-delayed time domain respiration signal including a plurality of successive sets of consecutive samples; performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample; performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples; determining a plurality of channelized instantaneous frequency values, each channelized instantaneous frequency value corresponding to a respective vector value; and remapping a power spectrum of the second FFT to a respective channelized instantaneous frequency value.

Para 3. The method of Para 1, further comprising: generating the time-delayed, time domain respiration signal from the acquired time domain respiration signal; and sampling the acquired time domain respiration signal and the time-delayed, time domain respiration signal.

Para 4. The method of Para 1, further comprising: generating the time-delayed, time domain respiration signal from the acquired time domain respiration signal; and sampling the acquired time domain respiration signal and the time-delayed, time domain respiration signal.

Para 5. The method of Para 4, further comprising acquiring the time domain respiration signal representative of the physiological parameter.

Para 6. The method of Para 5, further comprising filtering the one or more artifacts from the time domain respiration signal.

Para 7. The method of Para 6, further comprising displaying the filtered time domain respiration signal.

Para 8 The method of Para 6, wherein the method further comprises: determining that an alarm condition exists; and issuing an alarm.

Para 9. A physiological monitoring device, comprising: a processor; and a memory encoded with instructions that, when executed by the processor, performs a method comprising: performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

Para 10. The physiological monitoring device of Para 9, wherein the frequency domain analysis comprises: obtaining a time domain respiration signal representative of a physiological parameter and a time-delayed time domain respiration signal obtained from the acquired time domain respiration signal, each of the time domain respiration signal and the time-delayed time domain respiration signal including a plurality of successive sets of consecutive samples; performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample; performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples; determining a plurality of channelized instantaneous frequency values, each channelized instantaneous frequency value corresponding to a respective vector value; and

remapping a power spectrum of the second FFT to a respective channelized instantaneous frequency value.

Para 11. The physiological monitoring device of Para 9, the method further comprising: generating the time-delayed, time domain respiration signal from the acquired time domain respiration signal; and sampling the acquired time domain respiration signal and the time-delayed, time domain respiration signal.

Para 12. The physiological monitoring device of Para 11, the method further comprising acquiring the time domain respiration signal representative of the physiological parameter.

Para 13. The physiological monitoring device of Para 9, wherein: the physiological monitoring device further comprises a display; and the method further comprises: filtering the one or more artifacts from the acquired time domain respiration signal; and displaying the filtered time domain respiration signal.

Para 14. The physiological monitoring device of Para 9, wherein the method further comprises: determining that an alarm condition exists; and issuing an alarm.

Para 15. A patient monitoring system, comprising: a plurality of electrocardiogram ("ECG") leads over which a time domain respiration signal representative of a physiological parameter may be obtained; and a physiological monitoring device, comprising: a processor; and a memory encoded with instructions that, when executed by the processor, performs a method comprising: performing a frequency domain analysis of an acquired time domain respiration signal to identify one or more low frequency portions of the respiration signal that may be caused by one or more artifacts; and performing a time domain analysis of the respiration signal to modify thresholds during the one or more artifacts to compute a respiration rate from the time domain respiration signal.

Para 16. The patient monitoring system of Para 15, wherein the frequency domain analysis comprises: obtaining a time domain respiration signal representative of a physiological parameter and a time-delayed time domain respiration signal obtained from the acquired time domain respiration signal, each of the time domain respiration signal and the time-delayed time domain respiration signal including a plurality of successive sets of consecutive samples; performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample; performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples; determining a plurality of channelized instantaneous frequency values, each channelized instantaneous frequency value corresponding to a respective vector value; and remapping a power spectrum of the second FFT to a respective channelized instantaneous frequency value.

Para 17. The patient monitoring system of Para 15, the method further comprising: generating the time-delayed, time domain respiration signal from the acquired time domain respiration signal; and sampling the acquired time domain respiration signal and the time-delayed, time domain respiration signal.

Para 18. The patient monitoring system of Para 17, the method further comprising acquiring the time domain respiration signal representative of the physiological parameter.

Para 19. The patient monitoring system of Para 18, wherein: the physiological monitoring device further comprises a display; and the method further comprises: filtering the one or more artifacts from the acquired time domain respiration signal; and displaying the filtered time domain respiration signal.

Para 20. The patient monitoring system of Para 15, wherein the method further comprises: determining that an alarm condition exists; and issuing an alarm.

Para 21. The patient monitoring system of Para 15, further comprising a central monitoring station communicating with the physiological monitoring device.

Para 22. The patient monitoring system of Para 21, wherein the central monitoring system: receives the filtered time domain respiration signal from the physiological monitoring device; and displays the filtered time domain respiration signal.

Para 23. A method for use in monitoring the physical condition of a patient substantially as shown and described.

Para 24. A physiological monitoring device substantially as shown and described.

Para 25. A patient monitoring system substantially as shown and described.

**Claims**

1. A method for use in monitoring the physical condition of a patient, comprising:

    performing a frequency domain analysis of an acquired time domain respiration signal to identify one or more low frequency portions of the respiration signal that may be caused by one or more artifacts; and
    performing a time domain analysis of the respiration signal to modify thresholds during the one or more artifacts to compute a respiration rate from the time domain respiration signal.

2. The method of claim 1, wherein the frequency domain analysis comprises:

    obtaining the acquired time domain respiration and a time-delayed time domain respiration signal generated from the acquired time domain respiration signal, each of the acquired time domain respiration signal and the time-delayed time domain respiration signal including a plurality of successive sets of consecutive samples;
    performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample;
    performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples;
    determining a plurality of channelized instantaneous frequency values, each channelized instantaneous frequency value corresponding to a respective vector value; and
    remapping a power spectrum of the second FFT to a respective channelized instantaneous frequency value.

3. The method of claim 1 or 2, further comprising:

    generating the time-delayed, time domain respiration signal from the acquired time domain respiration signal; and
    sampling the acquired time domain respiration signal and the time-delayed, time domain respiration signal.

4. The method of claim 3, further comprising acquiring the time domain respiration signal representative of the physiological parameter.

5. The method of any one of the preceding claims, further comprising filtering the one or more artifacts from the time domain respiration signal, and optionally further comprising displaying the filtered time domain respiration signal.

6. The method of any one of the preceding claims, wherein the method further comprises:

    determining that an alarm condition exists; and
    issuing an alarm.

7. A physiological monitoring device, comprising:

    a processor; and
    a memory encoded with instructions that, when executed by the processor, performs a method comprising:

        performing a frequency domain analysis of an acquired time domain respiration signal to identify one or more low frequency portions of the respiration signal that may be caused by one or more artifacts; and
        performing a time domain analysis of the respiration signal to modify thresholds during the one or more artifacts to compute a respiration rate from the time domain respiration signal.

8. The physiological monitoring device of claim 7, wherein the frequency domain analysis comprises:

   obtaining a time domain respiration signal representative of a physiological parameter and a time-delayed time domain respiration signal obtained from the acquired time domain respiration signal, each of the time domain respiration signal and the time-delayed time domain respiration signal including a plurality of successive sets of consecutive samples;
   performing a Fast Fourier Transform ("FFT") for the acquired time domain respiration signal and for the time-delayed time domain respiration signal to obtain a first FFT for the time domain respiration signal and a second FFT for the acquired time domain respiration signal for each sample;
   performing a Nelson cross-spectral analysis of the first FFT and the second FFT for the acquired time domain respiration signal and the time-delayed time domain respiration signal to obtain a cross-spectral vector comprised of a plurality of vector values, each vector value corresponding to a respective one of the samples in the set of consecutive samples;
   determining a plurality of channelized instantaneous frequency values, each channelized instantaneous frequency value corresponding to a respective vector value; and
   remapping a power spectrum of the second FFT to a respective channelized instantaneous frequency value.

9. The physiological monitoring device of claim 8, the method further comprising:

   generating the time-delayed, time domain respiration signal from the acquired time domain respiration signal; and
   sampling the acquired time domain respiration signal and the time-delayed, time domain respiration signal.

10. The physiological monitoring device of claim 8 or 9, the method further comprising acquiring the time domain respiration signal representative of the physiological parameter.

11. The physiological monitoring device of any one of claims 7 to 10, wherein:

   the physiological monitoring device further comprises a display; and
   the method further comprises:

      filtering the one or more artifacts from the acquired time domain respiration signal; and
      displaying the filtered time domain respiration signal.

12. The physiological monitoring device of any one of claims 7 to 11, wherein the method further comprises:

   determining that an alarm condition exists; and
   issuing an alarm.

13. A patient monitoring system, comprising:

   a plurality of electrocardiogram ("ECG") leads over which a time domain respiration signal representative of a physiological parameter may be obtained; and
   the physiological monitoring device of any one of claims 7-12.

14. The patient monitoring system of claim 13, further comprising a central monitoring station communicating with the physiological monitoring device.

15. The patient monitoring system of claim 14, wherein the central monitoring system:

   receives the filtered time domain respiration signal from the physiological monitoring device; and
   displays the filtered time domain respiration signal.

FIG. 1

FIG. 2

*FIG. 3*

*FIG. 4*

500

```
┌─────────────────────────────────┐  ╭ 510
│      ACQUIRE A TIME DOMAIN        │
│       RESPIRATION SIGNAL          │
│     REPRESENTATIVE OF THE         │
│     PHYSIOLOGICAL PARAMETER       │
└─────────────────────────────────┘
                ⇓
┌─────────────────────────────────┐  ╭ 520
│   GENERATE A TIME-DELAYED, TIME   │
│    DOMAIN RESPIRATION SIGNAL      │
│     FROM THE ACQUIRED TIME        │
│    DOMAIN RESPIRATION SIGNAL      │
└─────────────────────────────────┘
                ⇓
┌─────────────────────────────────┐  ╭ 530
│     SAMPLE THE ACQUIRED TIME      │
│    DOMAIN RESPIRATION SIGNAL      │
│    AND THE TIME-DELAYED, TIME     │
│    DOMAIN RESPIRATION SIGNAL      │
└─────────────────────────────────┘
                ⇓
┌──────────────────────────────────────────────────┐  ╭ 535
│ PERFORM A FREQUENCY DOMAIN ANALYSIS OF THEN ACQUIRED │
│ TIME DOMAIN RESPIRATION SIGNAL TO IDENTIFY ONE OR MORE │
│ LOW FREQUENCY PORTIONS OF THE RESPIRATION SIGNAL THAT │
│     MAY BE CAUSED BY ONE OR MORE ARTIFACTS          │
└──────────────────────────────────────────────────┘
                ⇓
┌──────────────────────────────────────────────────┐  ╭ 540
│ PERFORM A TIME DOMAIN ANALYSIS OF THE RESPIRATION   │
│ SIGNAL TO MODIFY THRESHOLDS DURING THE ONE OR       │
│ MORE ARTIFACTS TO COMPUTE A RESPIRATION RATE        │
│ FROM THE TIME DOMAIN RESPIRATION SIGNAL             │
└──────────────────────────────────────────────────┘
```

## FIG. 5A

535

545

OBTAIN THE ACQUIRED TIME DOMAIN RESPIRATION AND THE TIME-DELAYED TIME DOMAIN RESPIRATION SIGNAL GENERATED FROM THE ACQUIRED TIME DOMAIN RESPIRATION SIGNAL. EACH OF THE ACQUIRED TIME DOMAIN RESPIRATION SIGNAL AND THE TIME-DELAYED TIME DOMAIN RESPIRATION SIGNAL INCLUDES A PLURALITY OF SUCCESSIVE SETS OF CONSECUTIVE SAMPLES

550

PERFORMING A FAST FOURIER TRANSFORM ("FFT") FOR THE ACQUIRED TIME DOMAIN RESPIRATION SIGNAL AND FOR THE TIME-DELAYED TIME DOMAIN RESPIRATION SIGNAL TO OBTAIN A FIRST FFT FOR THE TIME DOMAIN RESPIRATION SIGNAL AND A SECOND FFT FOR THE ACQUIRED TIME DOMAIN RESPIRATION SIGNAL FOR EACH SAMPLE

555

PERFORMS A NELSON CROSS-SPECTRAL ANALYSIS OF THE FIRST FFT AND THE SECOND FFT FOR THE ACQUIRED TIME DOMAIN RESPIRATION SIGNAL AND THE TIME-DELAYED TIME DOMAIN RESPIRATION SIGNAL TO OBTAIN A CROSS-SPECTRAL VECTOR COMPRISED OF A PLURALITY OF VECTOR VALUES, EACH VECTOR VALUE CORRESPONDING TO A RESPECTIVE ONE OF THE SAMPLES IN THE SET OF CONSECUTIVE SAMPLES

560

DETERMINE A PLURALITY OF CHANNELIZED INSTANTANEOUS FREQUENCY VALUES, EACH CHANNELIZED INSTANTANEOUS FREQUENCY VALUE CORRESPONDING TO A RESPECTIVE VECTOR VALUE

565

REMAP A POWER SPECTRUM OF THE SECOND FFT TO A RESPECTIVE CHANNELIZED INSTANTANEOUS FREQUENCY VALUE

FIG. 5B

FIG. 6

FIG. 8

*FIG. 7*

FIG.9

FIG.12

FIG. 10

EP 4 544 998 A1

FIG. 11

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FENG WANG ET AL: "Development of a PVDF Piezopolymer Sensor for Unconstrained In-Sleep Cardiorespiratory Monitori", JOURNAL OF INTELLIGENT MATERIAL SYSTEMS AND STRUCTURES, TECHNOMIC PUBL., LANCASTER, PA, US, vol. 14, no. 3, 1 January 2003 (2003-01-01), pages 185-190, XP009145176, ISSN: 1045-389X, DOI: 10.1177/1045389X03014003006 * page 187, left-hand column, last paragraph - page 189, left-hand column, paragraph 3 * * figures 3-6 * | 1-15 | INV. A61B5/08 A61B5/00 |
| A,D | NELSON DOUGLAS J: "Cross-spectral methods for processing speech", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 110, no. 5, 1 November 2001 (2001-11-01), pages 2575-2592, XP012002610, ISSN: 0001-4966, DOI: 10.1121/1.1402616 * page 2576, right-hand column, paragraph 3 - page 2580, right-hand column, paragraph 1 * | 1-15 | TECHNICAL FIELDS SEARCHED (IPC) A61B |
| A | US 2005/267362 A1 (MIETUS JOSEPH E [US] ET AL) 1 December 2005 (2005-12-01) * paragraph [0034] - paragraph [0063] * * figures 1-6 * | 1-15 | |

-/--

The present search report has been drawn up for all claims

| Place of search | Date of completion of the search | Examiner |
|---|---|---|
| Berlin | 16 January 2025 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**EUROPEAN SEARCH REPORT**

| Application Number |
| --- |
| EP 24 20 8805 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| A | US 2016/183846 A1 (DERKX RENE MARTINUS MARIA [NL]) 30 June 2016 (2016-06-30)<br>* paragraph [0008] *<br>* paragraph [0061] - paragraph [0065] *<br>* paragraph [0099] *<br>* paragraph [0106] - paragraph [0107] *<br>* figures 4,7,8 * | 1-15 | |
| A | US 2019/282180 A1 (BABAEIZADEH SAEED [US]) 19 September 2019 (2019-09-19)<br>* paragraph 50 - line 68, paragraph 60 *<br>* paragraph [0072] - paragraph [0074] *<br>* paragraph [0091] *<br>* paragraph [0094] *<br>* paragraph [0098] *<br>* figures 2,3,6 * | 1-15 | |

**TECHNICAL FIELDS SEARCHED (IPC)**

The present search report has been drawn up for all claims

| Place of search | Date of completion of the search | Examiner |
| --- | --- | --- |
| Berlin | 16 January 2025 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8805

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2025

| Patent document cited in search report | Publication date | Patent family member(s) | | Publication date |
|---|---|---|---|---|
| US 2005267362 A1 | 01-12-2005 | AU | 2006269263 A1 | 18-01-2007 |
| | | CA | 2619617 A1 | 18-01-2007 |
| | | DK | 1906817 T3 | 11-03-2019 |
| | | DK | 2526858 T3 | 10-06-2014 |
| | | EP | 1906817 A2 | 09-04-2008 |
| | | EP | 2526858 A1 | 28-11-2012 |
| | | ES | 2464151 T3 | 30-05-2014 |
| | | ES | 2716460 T3 | 12-06-2019 |
| | | JP | 5005687 B2 | 22-08-2012 |
| | | JP | 2009501060 A | 15-01-2009 |
| | | US | 2005267362 A1 | 01-12-2005 |
| | | US | 2010069762 A1 | 18-03-2010 |
| | | WO | 2007008706 A2 | 18-01-2007 |
| US 2016183846 A1 | 30-06-2016 | CN | 105451652 A | 30-03-2016 |
| | | EP | 3030148 A1 | 15-06-2016 |
| | | JP | 6430504 B2 | 28-11-2018 |
| | | JP | 2016535643 A | 17-11-2016 |
| | | US | 2016183846 A1 | 30-06-2016 |
| | | WO | 2015018752 A1 | 12-02-2015 |
| US 2019282180 A1 | 19-09-2019 | CN | 109414204 A | 01-03-2019 |
| | | EP | 3474739 A1 | 01-05-2019 |
| | | JP | 7019611 B2 | 15-02-2022 |
| | | JP | 2019524187 A | 05-09-2019 |
| | | US | 2019282180 A1 | 19-09-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NELSON, D**. Cross-Spectral Methods for Processing Speech. *J. Acoust. Soc. Am.*, 2001, vol. 110, 2575

- **FULOP, S**. An Accurate Means for Measuring Formants. *Dept. of Linguistics, Dept. of Computer Science*, 2003